# EUROPEAN PATENT SPECIFICATION

(11) **EP 3 267 896 B1**
(45) Date of publication and mention of the grant of the patent: **10.04.2024**
(21) Application number: 16709721.1
(22) Date of filing: 25.02.2016
(51) Int. Cl.: A61B 8/00, A61B 8/08, G06T 7/00, G06T 7/149, G16H 50/30

(54) **ULTRASONIC DIAGNOSIS OF CARDIAC PERFORMANCE BY SINGLE DEGREE OF FREEDOM CHAMBER SEGMENTATION**
ULTRASCHALLDIAGNOSE DER HERZLEISTUNG DURCH EINEN EINZIGEN KAMMERSEGMENTIERUNGSFREIHEITSGRAD
DIAGNOSTIC ULTRASONORE DE PERFORMANCES CARDIAQUES PAR UNE SEGMENTATION DE CHAMBRE À UN SEUL DEGRÉ DE LIBERTÉ

(30) Priority: 10.03.2015 US 201562130805 P; 10.03.2015 US 201562130787 P; 30.03.2015 EP 15161565
(43) Date of publication of application: 17.01.2018
(60) Divisional application: 23207853.5
(73) Proprietor: Koninklijke Philips N.V., 5656 AG Eindhoven (NL)
(72) Inventor: SCHNEIDER, Robert Joseph, 5656 AE Eindhoven (NL); PRATER, David, 5656 AE Eindhoven (NL); SETTLEMIER, Scott Holland, 5656 AE Eindhoven (NL); CARDINALE, Michael Daniel, 5656 AE Eindhoven (NL); BIANCHI, Mary Kay, 5656 AE Eindhoven (NL); RIVERA, Lydia, 5656 AE Eindhoven (NL); SALGO, Ivan, 5656 AE Eindhoven (NL)
(74) Representative: Philips Intellectual Property & Standards
(86) International application number: PCT/EP2016/054001
(87) International publication number: WO 2016/142183

(56) References cited:
- EP-A2- 2 434 454
- US-A- 5 601 084
- US-A1- 2009 136 109
- DIAS J M B ET AL: "WALL POSITION AND THICKNESS ESTIMATION FROM SEQUENCES OF ECHOCARDIOGRAPHIC IMAGES", IEEE TRANSACTIONS ON MEDICAL IMAGING, IEEE SERVICE CENTER, PISCATAWAY, NJ, US, vol. 15, no. 1, 2 February 1996 (1996-02-02), pages 25-38, XP000584331, ISSN: 0278-0062, DOI: 10.1109/42.481438

## Description

This invention relates to medical diagnostic ultrasound systems and, in particular, to the use of single degree of freedom cardiac chamber segmentation.

Ultrasonic imaging is routinely used to diagnose cardiac performance by measurement of parameters such as ejection fraction and cardiac output. Such measurements require the volume of blood in a heart chamber at various phases of the heart cycle to be delineated in two or three dimensional images of a heart chamber. Typically measurements of the volume of cardiac chambers, such as the left ventricle, have been generated by users hand tracing the endocardial boundary of the chamber. Such tracings are subject to significant variability due to differences in the criteria different users utilize in determining where to locate the tracing. Automatic methods have been developed to attempt to automate this boundary tracing, such as the automated border tracing methodology described in U.S. Pat. 6,491,636 (Chenal et al.) In this technique, anatomical landmarks of a chamber are located, including the mitral valve plane corners and the apex of the heart chamber. One of a plurality of standard, expert-validated endocardial shapes are then fit to these landmarks. The automatically drawn border can then be manually adjusted by rubberbanding, by which the user moves control points on the border to adjust its final position over the endocardium. This processing is done for images taken at the end systole and end diastole heart phases. The two borders can be compared or subtracted to estimate ejection fraction or cardiac output. For example, US 2009/0136109 discloses to produce a myocardium thickness volume by comparing the identified endocardial border (which defines an inner surface of the myocardium) and the identified epicardial border (which defines an outer surface of the myocardium). The myocardium thickness volume of US 2009/0136109 is hollow, with the hollow space inside being the volume of the heart chamber. Similar approach is described in EP 2434454 A2, wherein a volume of the left ventricle (LV) is computed based on the detected LV boundaries. The system of EP 2434454 A2 uses various machine learning for locating and tracking the cardiac wall. These training techniques based on the training data set, which uses a database of known cases collected for machine learning. Ejection fraction can be estimated by well-established methods such as an automated Simpson's algorithm (rule of disks), to measure the fraction of the chamber volume ejected with each contraction of the heart.

But automated image analysis methods do not always produce heart chamber delineations that are acceptable to all users. This lack of success is due in large part to the inability of the automatic methods to consistently locate the border where any given user believes the border should be placed. Much of this poor performance is due to variations between different users on what are the anatomical landmarks which specify where the true border lies.

It is an object of the present invention to provide users with a simple automated tool for delineating the location of an acceptable heart chamber border. It is a further object that such delineations be standardized, and capable of comparison and the production of repeatable results among different users. Such results summarize the approach of one clinician into a single value that can be understood and communicated to other clinicians who have the same tool.

These objects are solved by the independent claim. Advantageous embodiments are provided in the dependent claims.

In accordance with the principles of the present invention, a diagnostic ultrasound system according to independent claim 1 is disclosed, with optional features being presented in dependent claims 2 - 10.

In the drawings:
FIGURE 1 illustrates in block diagram form an ultrasonic diagnostic imaging system constructed in accordance with the principles of the present invention.
FIGURE 2 is a block diagram illustrating border detection details of the QLQB processor of FIGURE 1 in accordance with the principles of the present invention.
FIGURES 3a and 3b illustrate landmarks of the left ventricle which are useful for border detection.
FIGURES 4a, 4b and 4c illustrate expert-derived endocardial border shapes used for automated border detection.
FIGURES 5a and 5b illustrates the delineation of epicardial and endocardial borders in images of the left ventricle.
FIGURE 6 is a flowchart of the operation of a deformable heart model to find heart borders in accordance with the present invention.
FIGURES 7a and 7b are cardiac images of the left ventricle at end diastole and end systole in which the endocardial boundary and the interface between the trabeculaeted myocardium and the compacted myocardium have been traced.
FIGURES 8a and 8b illustrate the end systole ultrasound image of FIGURE 7b in which a user-defined border has been located at 0% and 100% of the distance between the two heart boundaries delineated in FIGURE 7b.
FIGURE 8c illustrates a cardiac image with a user-defined border is located at 40% of the distance toward the compacted myocardium interface from the endocardial tracing.
FIGURE 8d illustrates a single degree of freedom user control in accordance with the embodiments of the present invention, wherein the user-defined border is located relative to several boundaries of the myocardium.
FIGURE 9 illustrates user-defined heart chambers from biplane images which are being volumetrically measured using the rule of disks.
FIGURE 10 illustrates a user-defined heart chamber wire frame model from a 3D ultrasound image preparatory to volumetric measurement.

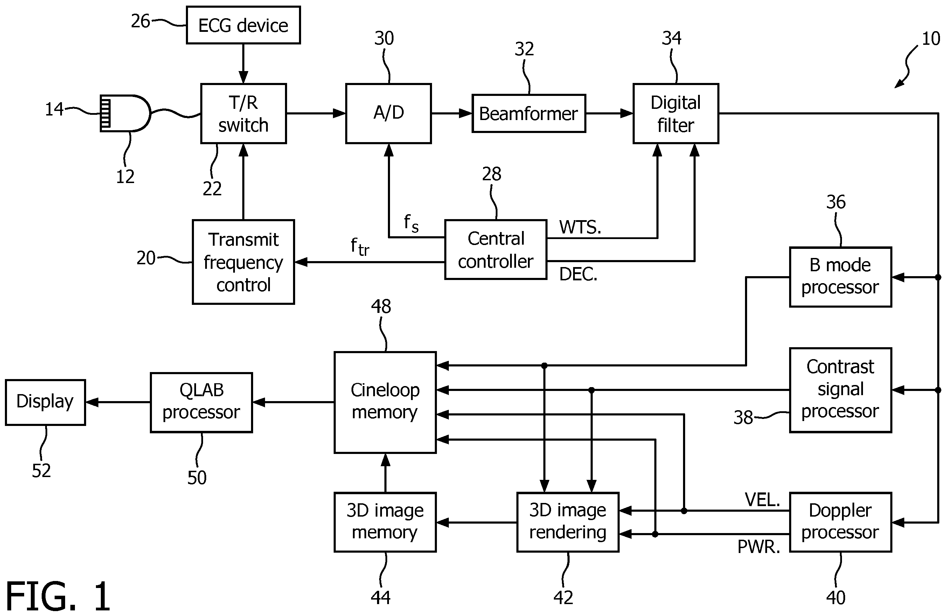

Referring first to FIGURE 1 an ultrasonic diagnostic imaging system 10 constructed in accordance with the principles of the present invention is shown in block diagram form. An ultrasonic probe 12 includes an array 14 of ultrasonic transducers that transmit and receive ultrasonic pulses. The array may be a one dimensional linear or curved array for two dimensional imaging, or may be a two dimensional matrix of transducer elements for electronic beam steering in three dimensions. The ultrasonic transducers in the array 14 transmit ultrasonic energy and receive echoes returned in response to this transmission. A transmit frequency control circuit 20 controls the transmission of ultrasonic energy at a desired frequency or band of frequencies through a transmit/receive ("T/R") switch 22 coupled to the ultrasonic transducers in the array 14. The times at which the transducer array is activated to transmit signals may be synchronized to an internal system clock (not shown), or may be synchronized to a bodily function such as the heart cycle, for which a heart cycle waveform is provided by an ECG device 26. When the heartbeat is at the desired phase of its cycle as determined by the waveform provided by ECG device 26, the probe is commanded to acquire an ultrasonic image. This enables acquisition at the end diastole and end systole heart phases, for instance. The frequency and bandwidth of the ultrasonic energy generated by the transmit frequency control circuit 20 is controlled by a control signal fₜᵣ generated by a central controller 28.

Echoes from the transmitted ultrasonic energy are received by the transducers in the array 14, which generate echo signals that are coupled through the T/R switch 22 and digitized by analog to digital ("A/D") converters 30 when the system uses a digital beamformer. Analog beamformers may also be used. The A/D converters 30 sample the received echo signals at a sampling frequency controlled by a signal fₛ generated by the central controller 28. The desired sampling rate dictated by sampling theory is at least twice the highest frequency of the received passband, and might be on the order of 30-40 MHz. Sampling rates higher than the minimum requirement are also desirable.

The echo signal samples from the individual transducers in the array 14 are delayed and summed by a beamformer 32 to form coherent echo signals. For 3D imaging with a two dimensional array, it is preferable to partition the beamformer between a microbeamformer located in the probe and the main beamformer in the system mainframe as described in US Pat. 6,013,032 (Savord) and US Pat. 6,375,617 (Fraser). The digital coherent echo signals are then filtered by a digital filter 34. In the illustrated ultrasound system, the transmit frequency and the receiver frequency are individually controlled so that the beamformer 32 is free to receive a band of frequencies which is different from that of the transmitted band such as a harmonic frequency band. The digital filter 34 bandpass filters the signals, and can also shift the frequency band to a lower or baseband frequency range. The digital filter can be a filter of the type disclosed in U.S. Patent No. 5,833,613, for example. Filtered echo signals from tissue are coupled from the digital filter 34 to a B mode processor 36 for conventional B mode image processing.

Filtered echo signals of a contrast agent, such as microbubbles, are coupled to a contrast signal processor 38. Contrast agents are often used to more clearly delineate the endocardial wall in relation to contrast agent in the blood pool of the heart chamber, or to perform perfusion studies of the microvasculature of the myocardium as described in US Pat. 6,692,438 for example. The contrast signal processor 38 preferably separates echoes returned from harmonic contrast agents by the pulse inversion technique, in which echoes resulting from the transmission of multiple differently modulated pulses to an image location are combined to cancel fundamental signal components and enhance harmonic signal components. A preferred pulse inversion technique is described in U.S. patent 6,186,950, for instance.

The filtered echo signals from the digital filter 34 are also coupled to a Doppler processor 40 for conventional Doppler processing to produce velocity and power Doppler signals. The output signals from these processors may be displayed as planar images, and are also coupled to a 3D image processor 42 for the rendering of three dimensional images, which are stored in a 3D image memory 44. Three dimensional rendering may be performed as described in U.S. patent 5,720,291, and in U.S. patents 5,474,073 and 5,485,842.

The signals from the contrast signal processor 38, the B mode processor 36 and the Doppler processor 40, and the three dimensional image signals from the 3D image memory 44 are coupled to a Cineloop^{®} memory 48, which stores image data for each of a large number of ultrasonic images. The image data are preferably stored in the Cineloop memory 48 in sets, with each set of image data corresponding to an image obtained at a respective time. The image data in a group can be used to display a parametric image showing tissue perfusion at a respective time during the heartbeat. The groups of image data stored in the Cineloop memory 48 may also be stored in a permanent memory device such as a disk drive or digital video recorder for later analysis. In this embodiment the images are also coupled to a QLAB processor 50, where the images are analyzed to automatically delineate borders of the heart, enabling a user to then position a border as the user believes most accurately indicates the true border of a chamber of the heart. The QLAB processor also makes quantified measurements of various aspects of the anatomy in the image and delineates tissue boundaries and borders by automated border tracing as described in US patent publication no. 2005/0075567 and PCT publication no. 2005/054898. The data and images produced by the QLAB processor are displayed on a display 52.

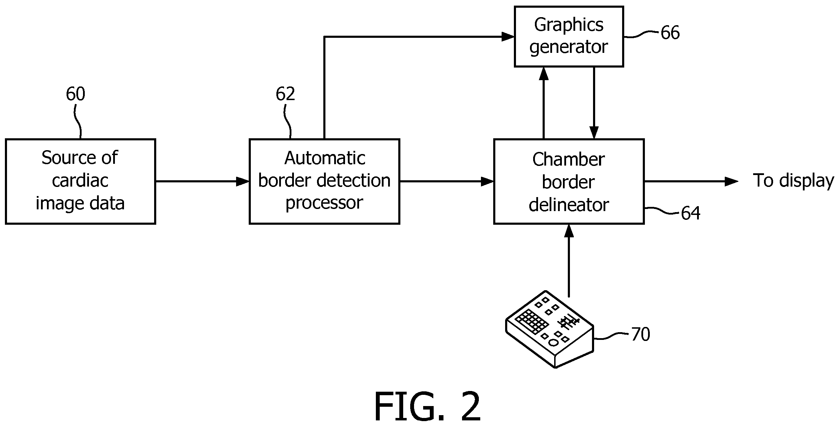

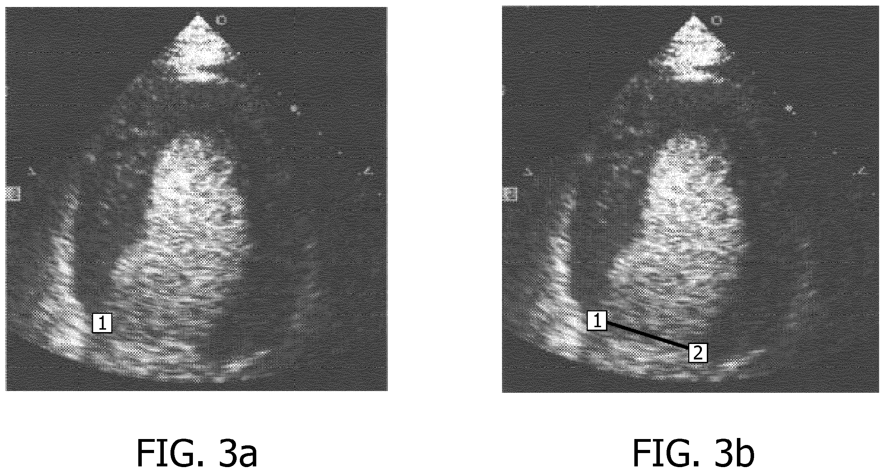

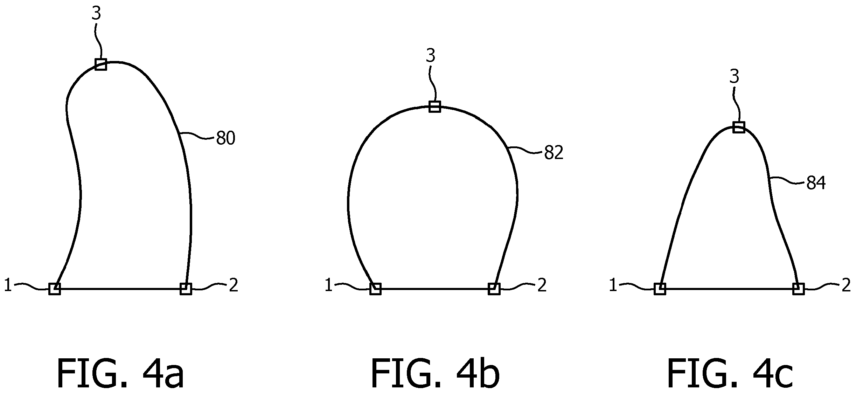

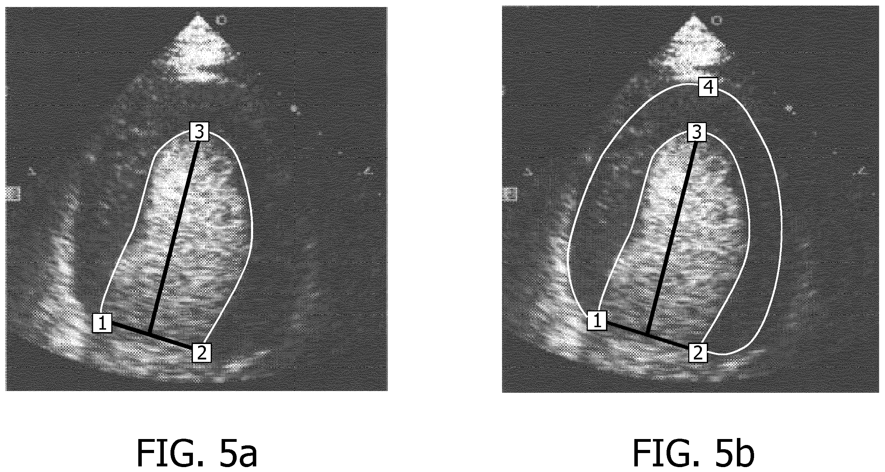

FIGURE 2 illustrates further details of the operation of the QLAB processor to delineate a user-defined heart chamber border in accordance with the principles of the present invention. A cardiac ultrasound image is provided by a source of cardiac image data 60, which may be the Cineloop memory 48, the 3D image memory 44, or one of image processors 36, 38, or 40 of FIGURE 1. The cardiac image is forwarded to an automatic border detection (ABD) processor 62. The ABD processor may be a fully automatic or a semi-automatic (user-assisted) image processor which delineates a border of a chamber in a heart image, several of which are described below. In a typical semi-automatic ABD system, the user designates a first landmark in the cardiac image with a pointing device such as a mouse or a trackball, usually located on the ultrasound system control panel 70 or with a workstation keyboard, which manipulates a cursor over the image. In the example of FIGURE 3a, for instance, the first landmark designated is the medial mitral annulus (MMA) at the bottom of the left ventricle (LV) in the illustrated view. When the user clicks on the MMA in the image, a graphic marker appears such as the white control point indicated by the number "1" in the drawing. The user then designates a second landmark, in this example, the lateral mitral annulus (LMA), which is marked with the second white control point indicated by the number "2" in FIGURE 3b. A line produced by the ABD processor then automatically connects the two control points, which in the case of this longitudinal view of the left ventricle indicates the mitral valve plane. The user then moves the pointer to the endocardial apex, which is the uppermost point within the left ventricular cavity. As the user moves the pointer to this third landmark in the image, a template shape of the left ventricular endocardial cavity dynamically follows the cursor, distorting and stretching as the user-manipulated pointer seeks the apex of the LV chamber, as shown in FIGURE 5a. This template, shown as a white line in FIGURE 5a, is anchored by the first and second control points 1 and 2 and passes through the third control point, which is positioned at the apex when the user clicks the pointer at the apex, positioning the third control point 3. Typical LV chamber border templates are shown in FIGURES 4a, 4b, and 4c. These templates are determined from many expert tracings of the LV endocardial boundary in many patients. The template 80 of FIGURE 4a is an elongated template typical of many normal patients. The template 82 of FIGURE 4b is more bulbous in shape, characteristic of many patients with congestive heart failure. The template 84 is yet a third possibility, a more teardrop shape. The template which best fits the three anatomical landmarks identified by the user is selected by the ABD processor 62 and distorted to fit the three user-defined landmarks. When positioned and fitted to the landmarks, the endocardial cavity template 80, 82, or 84 provides an approximate tracing of the endocardium of the LV, as shown in FIGURE 5a. In the example of FIGURE 5a a black line which bisects the left ventricle follows the pointer as it approaches and designates the apex. This black line is anchored between the center of the line indicating the mitral valve plane and the left ventricular apex, essentially indicating a center line between the center of the mitral valve and the apex of the cavity.

Once the ABD processor 62 has found the endocardial lining of the LV, it then attempts to find the epicardial boundary. This is illustrated in FIGURE 5b, where the user has moved the cursor and clicked on the apex 4 of the outside of the dark myocardium in the image. The images of FIGURE 5 are contrast-enhanced harmonic images in which the chamber of the LV has been flooded with a contrast agent but the agent has not yet fully perfused the myocardium, which is why the LV chamber appears very bright against the darker surrounding myocardium in this image. When the user clicks on the epicardial apex, the ABD processor, as before, selects an outer or epicardial template similar to the templates of FIGURE 4 and fits it to the epicardium as illustrated in FIGURE 5b. The cardiac image now has both its endocardial boundary (line connecting 1, 3 and 2 markers), the blood pool-myocardium interface, and its epicardial boundary (line connecting 1, 4 and 2 markers), the outmost surface of the heart, delineated in the image by tracings produced by a graphics generator 66.

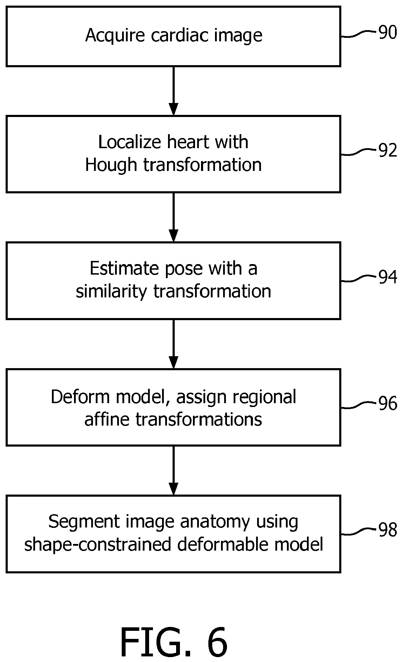

Instead of semi-automatic operation requiring user interaction, the ABD processor may delineate the border of the LV fully automatically as described in the aforementioned U.S. Pat. 6,491,636. As explained therein, an image processor can be configured to find the mitral valve corners and apex automatically, then fit a template to the automatically-located landmarks. However, a preferred technique for automatically delineating the myocardial borders is with a heart model, as illustrated in FIGURE 6. A heart model is a spatially-defined mathematical description of the tissue structure of a typical heart which can be fitted to the heart as it appears in a diagnostic image, thereby defining the specific anatomy of the imaged heart. The process of FIGURE 6 begins with the acquisition of a cardiac image at 90. The position of the heart is then localized in the cardiac image by processing the image data with a generalized Hough transform at 92. At this point the pose of the heart has not been defined, so misalignments in translation, rotation and scaling of the heart in the image data are corrected by use of a single similarity transformation for the whole heart model at 94. Next at 96, the model is deformed and affine transformations are assigned to specific regions of the heart. Constraints on the deformation are then relaxed by allowing the heart model to deform with respect to the piecewise affine transformation at 98, and the shape-constrained deformable model is resized and deformed so that each part of the model fits the actual patient anatomy as shown in the image at the captured phase of the heart cycle. The model is thus accurately adapted to the organ boundaries shown in the cardiac image, thereby defining the boundaries including the endocardial lining, the interface between the trabeculaeted myocardium and the compacted myocardium, and the epicardial border. In a preferred implementation of such a heart model, the interface between the trabeculaeted myocardium and the compacted myocardium is found first, as this typically appears as a well-defined gradient between a brightly illuminated region and a region of moderate illumination in an ultrasound image. The endocardial boundary is generally less well-defined in a heart model due to the desire to be able to find the variable location of the less well-defined endothelial lining as it appears in an ultrasound image. Unlike the contrast-enhanced cardiac images of FIGURES 5a and 5b, an unenhanced ultrasound image will generally exhibit a relatively sharp intensity gradient between the relatively high intensity echo tissue surrounding the myocardium and the medium intensity of the myocardium, and a relatively lesser gradient between the myocardium and the low intensity of the chamber's blood pool. This mandates in favor of discriminating the outer myocardial border first, then the inner endocardial boundary when diagnosing images acquired in the absence of a contrast agent. When the coordinates of a boundary have been found, they are communicated to the graphics generator 66, which generates the traces that overlie the image in the calculated positions.

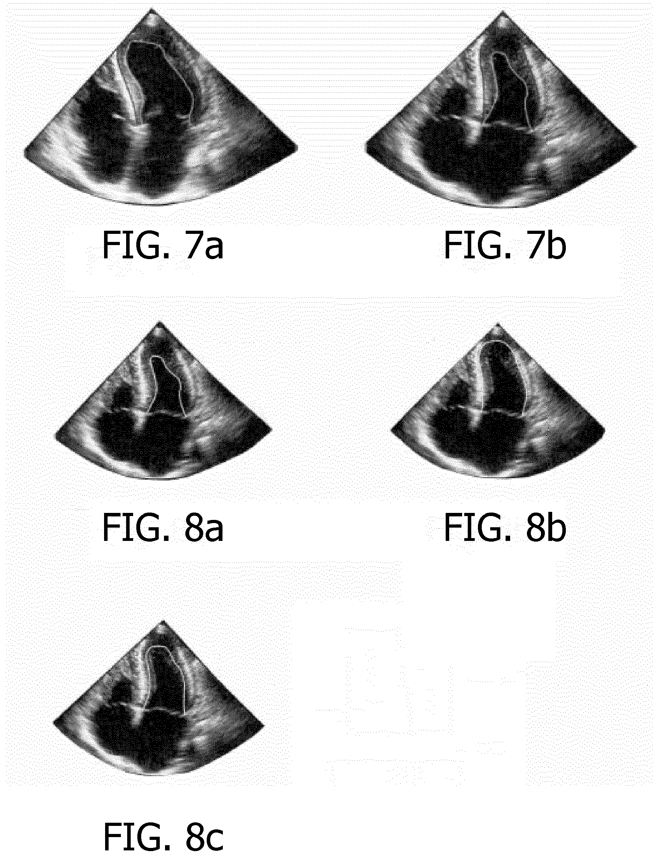

FIGURE 7 shows two ultrasound images, one with both boundaries of the myocardium outlined at end diastole (FIGURE 7a), and the second with both myocardial boundaries traced at end systole (FIGURE 7b). The compacted myocardial boundary is traced in black and the endocardial boundary is traced in white in these images. With both boundaries thus drawn, the user controls a chamber border delineator 64 with a user control on control panel 70 to indicate a location between the two tracings where the user believes the true chamber border is located. In one implementation the user operates a single variable control by which the user can position the true endocardial border at a location which is displaced a selected percentage of the distance between the previously drawn endocardial boundary and the previously drawn interface between the trabeculaeted myocardium and the compacted myocardium. FIGURE 8a illustrates the end systole image of FIGURE 7b when the single variable control is set at 0%, and FIGURE 8b illustrates the same image with the user control set at 100%, in which case the white line border is located at the outside of the myocardium. The visual tracing of the user-defined border is also produced by the graphics generator 66 in the system of FIGURE 1 for overlay over the ultrasound image. The user-set border is positioned at the called-for location as measured orthogonal to the endocardial tracing and at the called-for percentage of the distance between the two boundaries. In these examples the two myocardial boundary tracings are not shown for ease of illustration, which is one possible implementation, although the automatically drawn tracings could also be shown if desired.

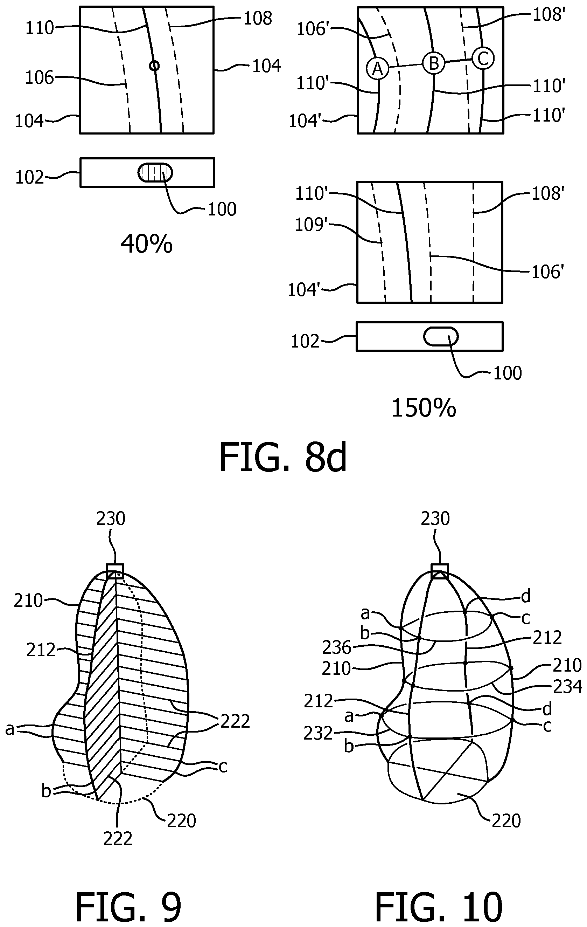

FIGURE 8c illustrates a situation in which the user has adjusted the user-positioned border (shown as a white line) so that it is 40% of the distance toward the compacted myocardium interface from the endocardial tracing. This is done by moving a slider 100 left or right in its slot 102. As the slider is manipulated by the user, the user-controlled border tracing 110 moves back and forth between the two boundaries of the myocardium. In the example of FIGURE 8c the slider 100 is shown as a softkey control on the display screen which is manipulated by a mouse or other user interface control, although the slide could alternatively be a physical slider, knob or switch, or a trackball on a conventional ultrasound system control panel. The user control can also be implemented as a rocker control, toggle buttons, list box, or a numerical entry box. If the user has a preferred percentage for most cases, this can be saved as a default value. In the example of FIGURE 8c the numerical percentage is displayed on the screen and changes as the slider 100 is moved. Also shown in FIGURE 8d in an enlarged view 104 of a portion of the identified myocardial boundaries. The user clicks on a point of the myocardium of the image to the left, and that portion of the myocardium appears in the enlarged view 104, with the user-manipulated border 110 shown between the myocardial boundaries 106 (outer boundary) and 108 (inner boundary). As the user manipulates the slider 100, the border 110 moves between the two system-drawn boundaries 106 and 108.

The user-manipulated border 110 can also be positioned using a range of more than 100% as shown in the enlarged view 104'. The user-defined position A (110'A) is positioned beyond the compacted myocardium boundary 106' and expressed in the range of more than 100%, while the user-defined position C (110'C) is positioned within the endocardial tracing 108' and expressed in negative percentage range. The user-defined position B (110'B) positioned in between the endocardial and compacted myocardium boundaries can be expressed in the range in between 0% and 100%.

Alternatively, for user convenience the user-manipulated (defined) border can be moved with respect to three outlines as illustrated in FIGURE 8d (lower to the right view 104'): the compacted myocardium boundary 106'and endocardial boundary 108', which correspond to the same percentage 100% and 0% respectively as in the previous example; and an additional boundary 109' corresponding to the percentage value of 200%, wherein the additional boundary 109' is located at the epicardial boundary. In the illustrated example, the slider 100 is adjusted at the value of 150% corresponding to the user-defined border 110' position in between the compacted myocardium boundary 106' and the epicardial boundary 109'. The alternative of the third boundary is addressing the fact that there is another boundary beyond the compacted myocardium boundary (106'), which is the epicardial boundary (denoted in this example as the additional boundary), said epicardial boundary may be also characterized by a noticeable and detectable gradient in the ultrasound image, which could be used to constrain the single degree of freedom slider. This epicardial boundary can be also identified by the border image processor. The slider is a single degree of freedom control. The user sets the position of the border 110 all around the chamber simply by setting the single value controlled by the slider. The value can be communicated to other users, who can obtain the same results by use of the same single numerical value.

FIGURES 9 and 10 illustrate how user-defined heart chamber borders of the present invention can be used to measure parameters such as cardiac output and ejection fraction. In the perspective view of FIGURE 9 two user-defined borders 210 and 212 of simultaneously acquired biplane images of the LV are shown on a base 220 which represents the mitral valve plane. The apex marker of the two borders is shown at 230. In this example the image planes of the two biplane image borders are orthogonal to each other. The volume within the two borders 210 and 212 is mathematically divided into spaced planes 222 which are parallel to the base plane 220. These planes intersect the left side of border 210 as shown at a,a and intersect the right side of border 210 as shown at c,c. The planes intersect the near side of tracing 212 as shown at b,b.

An ellipse is mathematically fit to the four intersection points a,b,c,d of each plane 222 as shown in FIGURE 9. While curves or splines other than ellipses can be used, including arcs and irregular shapes, an ellipse provides the advantage that Simpson's formula has been clinically validated when practiced with ellipses. The volume of the disks defined by the planes 222 and the ellipses can be calculated by the rule of disks to estimate the volume of the LV.

FIGURE 10 shows a wire frame model constructed of a user-defined border of a three dimensional heart chamber image. The horizontal sections 232, 234, 236 of the wire frame are border lines which intersect vertical border sections 210, 212 at intersection points a, b, c, and d. The horizontal sections are parallel to the mitral valve plane base 220. The volume within the wire frame can be determined by a modified rule of disks computation or other volumetric estimation technique. When a volume computed as shown in FIGURE 9 or 10 for an end systole phase image is subtracted from a volume calculated for an end diastole image and divided by the same, the result is an ejection fraction estimation.

Other variations of the above will readily occur to those skilled in the art. Instead of a percentage quantification, the user-defined border can be positioned an incremental distance from a manual or automatically traced boundary. The slider can be calibrated in distance so that the position of a user-defined border is a user-determined number of millimeters offset from a reference boundary, for instance. Rather than use two traced boundaries, the user-defined border can be located in reference to a single boundary tracing, or can be at an interpolated offset from more than two boundaries. The user-defined border can also be positioned using a range of more than 100%; it can be positioned within the endocardial tracing or beyond the epicardial or compacted myocardium boundary.

## Claims

1. An ultrasonic diagnostic imaging system (10) for determining the border of a chamber of the heart in an ultrasound image comprising:
a source of cardiac image data (60);
a border detection processor (62) responsive to the cardiac image data and arranged to identify at least an inner (106) and an outer (108) boundary of a myocardium in the image data;
a user control arranged to enable a user to adjust a single degree of freedom variable to indicate a user-defined border (110, 110', 110") of the heart chamber in relation to the inner and outer boundaries, wherein the single degree of freedom variable is calibrated in percentage in relation to the distance relative to the inner and outer boundaries, and wherein the percentage may be negative, or may be positive with a value between 0 and 100 or a value greater than 100; and
a chamber border delineator (64) coupled to the user control and the border detection processor, said chamber border delineator is arranged to locate the user-defined heart chamber border in the image data with respect to at least one of the boundaries identified by the border detection processor.

2. The ultrasonic diagnostic imaging system of Claim 1, wherein the border detection processor is arranged to identify in the cardiac image data an endocardium or a myocardium-blood pool interface as the inner boundary, and an epicardium or an interface between the trabeculaeted myocardium and the compacted myocardium as the outer boundary.

3. The ultrasonic diagnostic imaging system of Claim 1, wherein the user control further comprises a slider, a knob, a switch, a trackball, a rocker control, toggle buttons, a list box, or a numerical entry box.

4. The ultrasonic diagnostic imaging system of Claim 3, wherein the user control further comprises a softkey control or a physical control.

5. The ultrasonic diagnostic imaging system of Claim 1, wherein the source of cardiac image data further comprises a memory device containing two-dimensional cardiac images.

6. The ultrasonic diagnostic imaging system of Claim 5, wherein the cardiac images include a view of the left ventricle.

7. The ultrasonic diagnostic imaging system of Claim 1, wherein the border detection processor further comprises an automatic heart boundary image processor.

8. The ultrasonic diagnostic imaging system of Claim 7, wherein the automatic heart boundary image processor is further operable to identify a boundary of the myocardium in cardiac image data without user input.

9. The ultrasonic diagnostic imaging system of Claim 1, further comprising a graphics generator (66), coupled to the border detection processor, which is arranged to produce display traces of the inner and outer myocardium boundaries; and
a display (52), coupled to the source of cardiac image data and to the graphics generator, which is arranged to display a cardiac image with traced inner and outer myocardium boundaries.

10. The ultrasonic diagnostic imaging system of Claim 1, further comprising a graphics generator (66), coupled to the chamber border delineator, which is arranged to produce a display trace of the user-defined heart chamber border; and
a display (52), coupled to the source of cardiac image data and to the graphics generator, which is arranged to display a cardiac image with a user-traced heart chamber border.

## Patentansprüche

1. Ultraschall-Diagnosebildgebungssystem (10) zur Bestimmung der Grenze einer Herzkammer in einem Ultraschallbild, umfassend:
eine Quelle für Herzbilddaten (60);
einen Grenzerfassungsprozessor (62), der auf die Herzbilddaten reagiert und so angeordnet ist, dass er in den Bilddaten mindestens eine innere (106) und eine äußere (108) Grenze eines Myokards identifiziert;
eine Benutzersteuerung, die so angeordnet ist, dass sie es einem Benutzer ermöglicht, eine einzelne Freiheitsgradvariable anzupassen, um eine benutzerdefinierte Grenze (110, 110', 110") der Herzkammer in Bezug auf die inneren und äußeren Grenzen anzuzeigen, wobei die einzelne Freiheitsgradvariable in Prozent in Bezug auf den Abstand relativ zu den inneren und äußeren Grenzen kalibriert wird und wobei der Prozentsatz negativ oder positiv mit einem Wert zwischen 0 und 100 oder einem Wert größer als 100 sein kann; und
einen Kammergrenze-Abgrenzer (64), der mit der Benutzersteuerung und dem Grenzerfassungsprozessor gekoppelt ist, wobei der Kammergrenze-Abgrenzer so angeordnet ist, dass er die vom Benutzer definierte Herzkammergrenze in den Bilddaten in Bezug auf mindestens eine der vom Grenzerfassungsprozessor identifizierten Grenzen lokalisiert.

2. Ultraschall-Diagnosebildgebungssystem nach Anspruch 1, wobei der Grenzerfassungsprozessor so angeordnet ist, dass er in den Herzbilddaten ein Endokard oder eine Myokard-Blut-Pool-Grenzfläche als innere Grenze und ein Epikard oder eine Grenzfläche zwischen dem Trabekel-Myokard und dem verdichteten Myokard als äußere Grenze identifiziert.

3. Ultraschall-Diagnosebildgebungssystem nach Anspruch 1, wobei die Benutzersteuerung außerdem einen Schieber, einen Knopf, einen Schalter, einen Trackball, eine Wippsteuerung, Umschalttasten, ein Listenfeld oder ein numerisches Eingabefeld umfasst.

4. Ultraschall-Diagnosebildgebungssystem nach Anspruch 3, wobei die Benutzersteuerung außerdem eine Softkey-Steuerung oder eine physische Steuerung umfasst.

5. Ultraschall-Diagnosebildgebungssystem nach Anspruch 1, wobei die Quelle der Herzbilddaten außerdem eine Speichervorrichtung umfasst, die zweidimensionale Herzbilder enthält.

6. Ultraschall-Diagnosebildgebungssystem nach Anspruch 5, wobei die Herzbilder eine Ansicht des linken Ventrikels umfassen.

7. Ultraschall-Diagnosebildgebungssystem nach Anspruch 1, wobei der Grenzerfassungsprozessor außerdem einen automatischen Herzgrenzbildprozessor umfasst.

8. Ultraschall-Diagnosebildgebungssystem nach Anspruch 7, wobei der automatische Herzgrenzen-Bildprozessor außerdem dazu dient, eine Grenze des Myokards in Herzbilddaten ohne Benutzereingabe zu identifizieren.

9. Ultraschall-Diagnosebildgebungssystem nach Anspruch 1, das außerdem einen Grafikgenerator (66) umfasst, der mit dem Grenzerfassungsprozessor gekoppelt ist und angeordnet ist, um Anzeigespuren der inneren und äußeren Myokardgrenzen zu erzeugen; und eine Anzeige (52), die mit der Quelle der Herzbilddaten und dem Grafikgenerator verbunden ist und angeordnet ist, um ein Herzbild mit nachgezeichneten inneren und äußeren Myokardgrenzen anzuzeigen.

10. Ultraschall-Diagnosebildgebungssystem nach Anspruch 1, das außerdem einen Grafikgenerator (66) umfasst, der mit dem Kammergrenze-Abgrenzer gekoppelt ist und so angeordnet ist, dass er eine Anzeigespur der benutzerdefinierten Herzkammergrenze erzeugt; und eine Anzeige (52), die mit der Quelle der Herzbilddaten und dem Grafikgenerator verbunden ist und angeordnet ist, um ein Herzbild mit einer vom Benutzer nachgezeichneten Herzkammergrenze anzuzeigen.

## Revendications

1. Système d'imagerie diagnostique ultrasonique (10) pour déterminer la limite d'une chambre du coeur dans une image ultrasonore comprenant:
une source de données d'image cardiaque (60);
un processeur de détection de limites (62) en réponse aux données d'image cardiaque et agencé pour identifier au moins une délimitation interne (106) et une délimitation externe (108) d'un myocarde dans les données d'image;
une commande d'utilisateur agencée pour permettre à un utilisateur d'ajuster une variable à un seul degré de liberté pour indiquer une limite définie par l'utilisateur (110, 110', 110") de la chambre du coeur par rapport aux délimitations interne et externe, où la variable à un seul degré de liberté est calibrée en pourcentage par rapport à la distance par rapport aux délimitations interne et externe, et où le pourcentage peut être négatif, ou peut être positif avec une valeur comprise entre 0 et 100 ou une valeur supérieure à 100; et
un délimiteur de limite de la chambre (64) couplé à la commande d'utilisateur et au processeur de détection de limites, ledit délimiteur de limite de la chambre est agencé pour localiser la limite de la chambre du coeur définie par l'utilisateur dans les données d'image par rapport à au moins une des délimitations identifiées par le processeur de détection de limites.

2. Système d'imagerie diagnostique ultrasonique selon la revendication 1, dans lequel le processeur de détection de limites est agencé pour identifier dans les données d'image cardiaque un endocarde ou une interface myocarde-pool sanguin comme la délimitation interne, et un épicarde ou une interface entre le myocarde trabéculé et le myocarde compacté comme la délimitation externe.

3. Système d'imagerie diagnostique ultrasonique selon la revendication 1, dans lequel la commande d'utilisateur comprend en outre un curseur, un bouton, un interrupteur, une boule de commande, une commande à bascule, des boutons à bascule, une zone de liste ou une zone de saisie numérique.

4. Système d'imagerie diagnostique ultrasonique selon la revendication 3, dans lequel la commande d'utilisateur comprend en outre une commande par touche logicielle ou une commande physique.

5. Système d'imagerie diagnostique ultrasonique selon la revendication 1, dans lequel la source de données d'image cardiaque comprend en outre un dispositif de mémoire contenant des images cardiaques bidimensionnelles.

6. Système d'imagerie diagnostique ultrasonique selon la revendication 5, dans lequel les images cardiaques comprennent une vue du ventricule gauche.

7. Système d'imagerie diagnostique ultrasonique selon la revendication 1, dans lequel le processeur de détection de limites comprend en outre un processeur d'image de délimitation du coeur automatisé.

8. Système d'imagerie diagnostique ultrasonique selon la revendication 7, dans lequel le processeur d'image de délimitation du coeur automatisé sert également à identifier une délimitation du myocarde dans des données d'image cardiaque sans des entrées de l'utilisateur.

9. Système d'imagerie diagnostique ultrasonique selon la revendication 1, comprenant en outre un générateur graphique (66), couplé au processeur de détection de limites, qui est agencé pour produire des traces d'affichage des délimitations interne et externe du myocarde; et
un afficheur (52), couplé à la source de données d'image cardiaque et au générateur graphique, qui est agencé pour afficher une image cardiaque avec des délimitations du myocarde interne et externe tracées.

10. Système d'imagerie diagnostique ultrasonique selon la revendication 1, comprenant en outre un générateur graphique (66), couplé au délimiteur de limite de la chambre, qui est agencé pour produire une trace d'affichage de la limite de la chambre du coeur définie par l'utilisateur; et
un afficheur (52), couplé à la source de données d'image cardiaque et au générateur graphique, qui est agencé pour afficher une image cardiaque avec une limite de la chambre du coeur tracée par l'utilisateur.
